# EUROPEAN PATENT APPLICATION

(11) **EP 2 749 553 A1**
(43) Date of publication of application: **02.07.2014**
(21) Application number: 12199791.0
(22) Date of filing: 31.12.2012
(51) Int. Cl.: C07D 205/08, C07C 213/06, C07C 217/54

(54) **Process for preparation of enantiomerically pure S-(+)-N, N-dimethyl-a-[2-(naphthalenyloxy)ethyl] benzenemethanamine**

(71) Applicant: Deva Holding Anonim Sirketi, 34303 Istanbul (TR)
(72) Inventor: Koc, Fikret, 34303 Istanbul (TR); Karliga, Bekir, 34303 Istanbul (TR); Bellur Atici, Esen, 34303 Istanbul (TR); Ilhan, Suna Ayse, 34303 Istanbul (TR)

(57) **Abstract**

The present invention relates to improved, efficient process for the preparation of enantiomerically pure S-(+)-N,N-dimethyl-a-[2-(naphthalenyloxy)ethyl] benzenemethanamine and pharmaceutically acid addition salts thereof. The present invention particularly provides a process for preparation of (3S, 4R)-3-hydroxy-1-(4-methoxyphenyl)-4-phenylazetidin-2-one useful as a key intermediate for preparation of (s)-dapoxetine.

## Description

### Technical Field

The present invention relates to improved, efficient process for the preparation of enantiomerically pure S-(+)-N,N-dimethyl-a-[2-(naphthalenyloxy)ethyl] benzenemethanamine and pharmaceutically acid addition salts thereof. The present invention particularly provides a process for preparation of (3S, 4R)-3-hydroxy-1-(4-methoxyphenyl)-4-phenylazetidin-2-one useful as a key intermediate for preparation of (s)-dapoxetine.

### Background Art

Premature ejaculation is the most common form of ejaculatory dysfunction, affecting almost third of male population. The most advance agent for premature ejaculation is selective serotonin reuptake inhibitors (SSRIs).

Dapoxetine is selective serotonin reuptake inhibitor (SSRI) with a short life, developed specifically for the treatment of men with premature ejaculation (PE). Dapoxetine differs from existing SSRIs in having a unique pharmacokinetic profile, with a short initial plasma half life (about 1 h) and rapid elimination (initial half-life of 1-2 h).

Dapoxetine is chemically known as S-(+)-N, N-dimethyl-a-[2-(naphthalenyloxy) ethyl] benzenemethanamine. As a free base, dapoxetine has the problem of poor in vivo solubility which leads to poor water solubility and low in vivo absorption.

As the poor water solubility makes it difficult to process in solid oral dosage forms, dapoxetine has been developed in the form of hydrochloride salt and it is represented by the following formula;

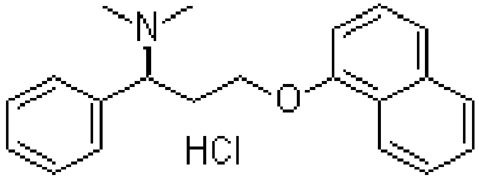

In the course of investigations of both enantiomers, the clinical studies have shown that the (S)-enantiomer of dapoxetine is 3.5 times more potent than (R)-enantiomer. Due to its superior pharmacological activity, asymmetric synthetic approaches and separation of enantiomers for obtaining pure (S)-dapoxetine have been developed lately.

There are only a few reported routes synthesis for (s)-dapoxetine in the literature.

EP 0288188 B (ELI LILLY AND COMPANY) 16.10.1991 discloses firstly 1-phenyl-3-naphtalenoxypropanamines and their use in inhibiting serotonin uptake. It described the process for the preparation by reacting 1-halo-1-phenyl-propylamine compound with amine. The '188 patent also discloses the use of dibenzoyl-d-and-1-tartaric acid to obtain enantiomerically pure dapoxetine and its hydrochloride salt.

WO 2008/035358 A (CADILLA HEALTHCARE LIMITED) 27.03.2008 discloses a process for preparation of enantiomerically pure dapoxetine, by resolving racemic (±)-dapoxetine with a chiral acid so as to obtain salt of the chiral acid and (+)-dapoxetine, substantially free from (-)-dapoxetine.

WO 2011/161690 A (SYMED LABS LIMITED) discloses a process for preparation of the compound 3(S)-(+)-N,N-dimethylamino-3-phenyl propanol as intermediate in the synthesis of S(+)-N,N-dimethyl-2-[1-(naphthalenyloxy)ethyl]benzene methanamine and intermediates thereof.

VENKATESAN, K, et al. A Stereoselective Synthesis of (S)-dapoxetine starting from trans-cinnamyl alcohol. ARKAT USA. 2008, p.302-310. describes stereoselective synthesis of (s)-(+)-dapoxetine from trans-methyl cinnamate and cinnamyl alcohol, employing sharpless asymmetric dihydroxylation and epoxidation process.

The last mentionned processes which are reported in the prior art are quite long and they require a radical deoxygenation step to remove the undesired hydroxyl group.

As the multiple steps, lengthy procedures and costly reagents increase production costs, there is a need for the development of an improved, simple, efficient method of (s)-dapoxetine synthesis with better yield. Moreover, higher stereoselective synthesis is highly desirable in (s)-dapoxetine synthesis.

### Summary of invention

The present invention relates to improved, efficient process for the preparation of enantiomerically pure S-(+)-N,N-dimethyl-a-[2-(naphthalenyloxy)ethyl] benzenemethanamine. Particularly, the present invention provides a process for preparation of (3S, 4R)-3-hydroxy-1-(4-methoxyphenyl)-4-phenylazetidin-2-one which is useful as a key intermediate for preparation of (s)-dapoxetine.

### Technical Problem

In spite of important advances in asymmetric synthesis, industrial scale production of enantiomerically pure (s)-dapoxetine is not all along practicable and resolution of enantiomers becomes indispensable.

The commercial production of (S)-dapoxetine which relies on the tartaric acid, promoted chiral resolution of racemic dapoxetine.

Inventors distinguished very few reported methods for synthesis of (S)-dapoxetine, but they all raise difficulties in terms of repeatability and stereoselectivity of the reaction and lead to irreproducible results with poor yield and quality.

There is a need to develop a process which is easy to commercialize and which yields in good quality as well as quantity.

### Solution to Problem

The present invention is designed to solve existing problems of the prior art and it is an object of the present invention to develop an improved process for preparation of enantiomerically pure (s)-dapoxetine.

In the present invention, inventors investigated a route of synthesis with fewer steps and much higher overall yield for the preparation of (s)-dapoxetine.

As it is considered as a key intermediate in the preparation of S-(+)-N,N-dimethyl-a-[2-(naphthalenyloxy)ethyl] benzenemethanamine and pharmaceutically acceptable salts, the synthesis of (3S, 4R)-3, hydroxy-1-(4-methoxyphenyl)-4-phenylazetidin-2-one is primarly intented by inventors.

For this purpose, inventors of the present invention have obtained racemic substituted azetidin-4-one derivative and surprisingly found that the latter can be separated to its enantiomers via lipase-catalyzed kinetic resolution.

Obtained enantiomerically pure intermediate (3S, 4R)-3, hydroxy-1-(4-methoxyphenyl)-4-phenylazetidin-2-one provided an enantioselective, simple process for preparation (s)-dapoxetine and pharmaceutically acceptable salts thereof.

The foregoing process has been found to be markedly attractive in both commercial and manufacturing view and affords good quality of (s)-dapoxetine.

Described invention is an industrially advantageous, reproducible, robust, eco-friendly, inexpensive amenable to scale up process for the preparation of (s)-dapoxetine and a pharmaceutically acceptable salt thereof.

### Description of embodiments

The present invention relates to an improved process for the preparation of S-(+)-N, N-dimethyl-a-[2-(naphthalenyloxy) ethyl] benzenemethanamine or a pharmaceutically acceptable salt thereof and intermediates.

Based on retrosynthetic strategy, our inventors have considered (3S, 4R)-3, hydroxy-1-(4-methoxyphenyl)-4-phenylazetidin-2-one as a potential block for the total synthesis of (+)-(S)-dapoxetine.

The present invention relates to the process for the preparation of S-(+)-N, N-dimethyl-a-[2-(naphthalenyloxy) ethyl] benzenemethanamine and pharmaceutically acceptable salts thereof from (3S, 4R)-3, hydroxy-1-(4-methoxyphenyl)-4-phenylazetidin-2-one.

In a first aspect of the present invention, the process comprises the following steps:

(a) reacting benzaldehyde(I) with *p*-anisidine(II) to form the following imine of formula (III).

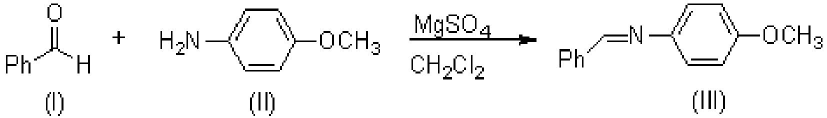

(b) reacting N-(4-methoxyphenyl) benzaldimine (III) with acetoxyacetyl chloride(IV) in presence of triethylamine/dichloromethane to provide racemic 4-aryl substituted β-lactam derivative of formula (V) in high yield.

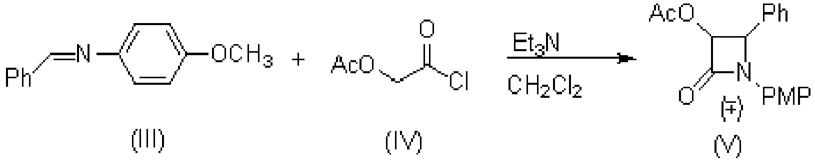

The enzyme catalysed resolution of enantiomers of formula (V) is investigated by inventors to obtain the desired isomer. The lipase mediated hydrolysis is found the best fit for enantiomer separation.

(c) converting intermediate of formula(V) (±), namely 1-(p-methoxyphenyl-3-acetoxy-4-phenylazetidin-2-one into its single enantiomer product via lipase-enzyme catalysed resolution, as follows:

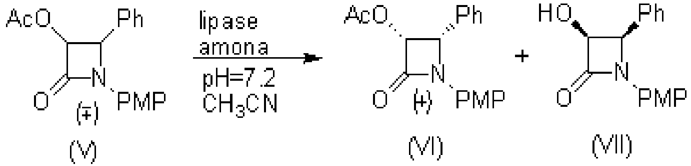

The racemic 4-aryl substituted β-lactam derivative of formula (V) is separated into enantiomers by subjection to lipase-PS catalyzed hydrolysis in phosphate buffer (pH=7.2).

Lipase is an enzyme acting on carboxylic esters, and classified as hydrolase, sub-classed in (EC.3.1.1) by E.C. Enzyme Commission of International Union of Biochemistry and Molecular Biology.

Lipases, namely one of triacylglycerol acyl hydrolases, are known to be used in wide applications ranging from enantioselective syntheses and resolution of racemic mixtures.

In the present invention, inventors used lipase-enzyme catalysed resolution to obtain enantiomerically pure (3S, 4R)-3-hydroxy-1-(4-methoxyphenyl)-4-phenylazetidin-2-one (VII) for the first time in preparation of (s)-dapoxetine. The completion of the reaction is monitored by TLC.

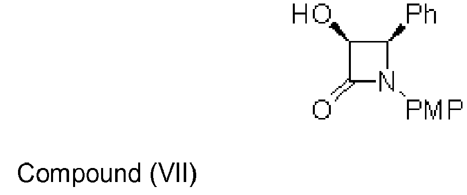

As developed method contains fewer steps than traditional route, inventors attempted to use obtained enantiomerically pure intermediate in the preparation of (s)-dapoxetine for the first time.

The high yield and less number of steps render our approach a good alternative to known methods in the preparation (3S, 4R)-3-hydroxy-1-(4-methoxyphenyl)-4-phenylazetidin-2-one.(VII)

(d)converting, the 3-hydroxy b-lactam derivative(VII) to its xanthate derivative(VIII)

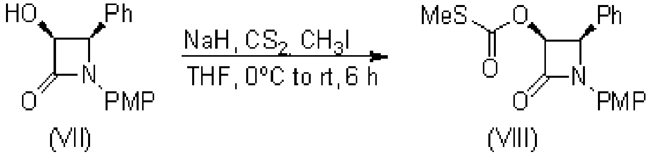

(e) subjecting xanthate derivative of formula (VIII) to deoxygenation to furnish β-lactam of formula (IX).

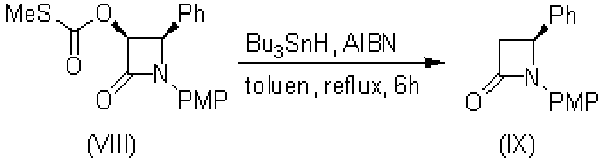

(f) subjecting lactam's p-methoxy-phenyl group of β-lactam to oxidative removal to obtain the compound (X).

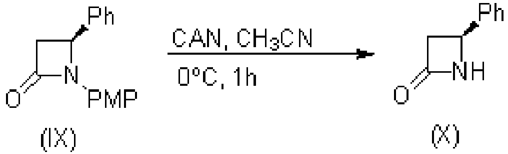

(g) protecting lactam (X) as its carbamate derivative with (BOC)₂O to form the compound(XI).

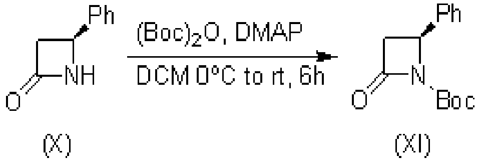

(h) reducting of the compound (XI)by LAH to get BOC protected amino alcohol, namely compound (XII).

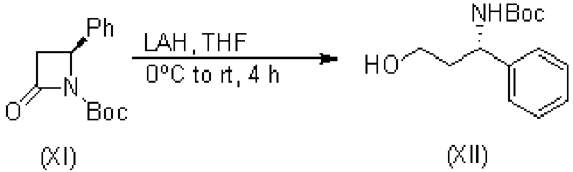

(i),deprotecting of the compound (XII) with TFA to get amino-alcohol compound (XIII).

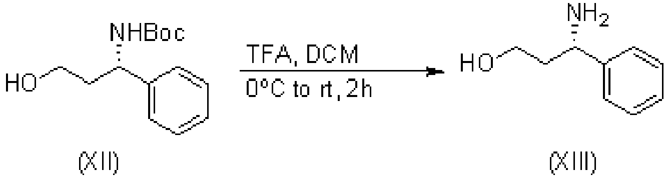

(j) demethylating of the compound (XIII) by using of formaldehyde and sodium cyanoborohydride afforded the desired N- bismethylated amino alcohol of formula (XIV).

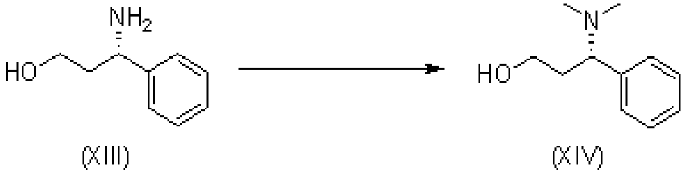

(k) obtaining of enantiomerically pure (s)-dapoxetine by the following reaction.

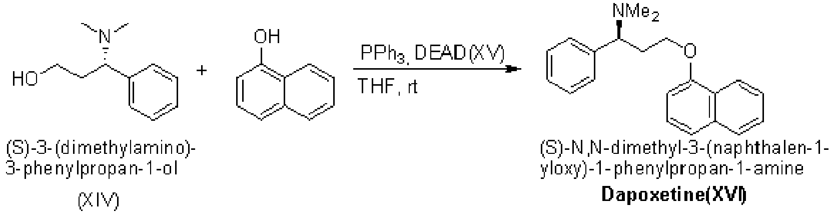

In another aspect of the present invention, the characterization of obtained intermediates is done by NMR spectroscopy.¹H and ¹³C experiments were recorded on Mercury-300 NMR spectrometer (¹H, 300.13 MHz and ¹³C, 75.5MHz).

Furthermore, the melting points were measured on an electrothermal 9100 apparatus.

**Compound( III):**

**The 1H-NMR** (300MHz, CDCl₃) of compound (III) showed peaks as δ 8.41 (s, 1H), 57.66-7.56 (m, 3H), 57.32-7.28 (m, 1H), 57.22-7.18 (m, 2H), δ6.93-6.91(m, 2H), δ) 3.81 (s, 3H).

**The 13C-NMR** (CDCl₃) of compound (III) showed peaks as δ 156.28, δ140.82, δ29.78, δ129.12, δ128.85, δ128.12, δ124.45, δ121.60, 5114.44, δ111.75, δ54.82.

**Compound (V) :**

The**1H-NMR** (300MHz, CDCl₃) of compound (V) showed peaks as δ 7.43-7.28 (m, 5H), δ 7.18-7.15 (m, 2H), 57.08-7.05 (m, 2H), δ 5.22 (d, J=5.2 Hz, 1H), δ 5.13 (dd, J=5.2, 9.8 Hz, 1H), 53.74 (s, 3H), 51.92 (s, 3H).

**Compound( VII):**

**The 1H-NMR** (300MHz, CDCl₃) of compound (VII) showed peaks δ 7.43-7.28 (m, 5H), δ7.18-7.15 (m, 2H), 57.08-7.05 (m, 2H), δ 5.22 (d, J=5.2 Hz, 1H), 55.13 (dd, J=5.2, 9.8 Hz, 1H), 53.74 (s, 3H), 53.6 (bs, OH, 1H).

**Compound( VIII):**

**The 1H-NMR** (300MHz, CDCl₃) of compound (VIII) showed peaks as δ 7.37-7.27 (7H, m, Ar), δ6.81 (2H, d, J¼8.9 Hz, Ar ), δ6.68 (1H, d, J¼4.8 Hz, CH-OC]S), 55.42 (1H, d,J¼4.8 Hz, CH-N), 53.76 (3H, s, OCH3), 52.29 (3H, s, SCH3).

**The 13C-NMR** (75.5 MHz, CDCl₃) of compound (VIII)showed peaks as 5 213.6, δ160.5, δ156.5, δ131.8, δ130.0, δ128.7, δ128.3, δ128.1, δ118.8, δ114.3, δ81.6,δ 61.6, δ55.3, δ18.8.

**Compound(IX):**

**The 1H-NMR** (300MHz, CDCl₃) of compound (IX) showed peaks as 5 7.28-7.13 (7H, m, Ar), 56.69 (2H, d, J¼8.9 Hz, Ar),δ 4.90-4.86 (1H, m, CH-N),5 3.64 (3H, s, OCH3), 53.45 (1H, dd, J¼5.5, 15 Hz, OCCH2 0), 52.83 (1H, dd, J¼2.5, 15 Hz, OCCH).

**The 13C-NMR** (75.5 MHz, CDCl₃) of compound (IX) showed peaks as δ 163.9, δ155.8, δ138.2,δ131.3, δ129.0, δ128.4,δ125.8,δ118.0,δ114.1, δ55.3, δ53.9, δ46.8, δ4.5.

**Compound(X):**

**The 1H-NMR** (300MHz, CDCl₃) of compound (X) showed peaks as δ_{H} 7.30-7.19 (5H, m, Ar), 56.23 (1H, br s, NH), 54.66 (1H, m, CH-N), 53.44-3.32 (1H, m, OCCH2 0), 52.81 (1H, dd, J¼2.09, 14.09 Hz, OCCH2);

**The 13C-NMR** (75.5 MHz, CDCl₃) of compound (X) showed peaks as δ167.9, δ140.2, δ128.4, δ127.6, δ125.3, δ49.7, δ 47.4.

**Compound(XI):**

**The 1H-NMR** (300MHz, CDCl₃) of compound (XI) showed peaks as δ_{H} 7.36-7.25 (5H, m, Ar),δ 4.85 (1H, m, CH-N), 53.36 (1H, dd, J¼6.1, 16 Hz, OCCH₂ 0), 52.85 (1H, dd, J¼3.15, 16 Hz, OCCH2), 51.31 (9H, s, OC(CH₃ )₃);

**The 13C-NMR** (75.5 MHz, CDCl₃) of compound (XI) showed peaks as δ 164.9, δ147.3, δ138.1, δ128.7, δ128.4, δ125.8, δ83.1, δ53.6, δ45.9, δ27.7.

**Compound(XII):**

**The 1H-NMR** (300MHz, CDCl₃) of compound (XII) showed peaks as δ_{H} 7.38-7.27 (5H, m, Ar), 54.99 (1H, m, CH-N), δ3.72-3.65 (2H, m, OCH₂), 2.7 (2H, br s, OH, NH), 52.17-1.79 (2H, m, CCH₂C), 51.44 (9H, s, OC (CH ₃)₃).

**The 13C-NMR** (75.5 MHz, CDCl₃) of compound (XII) showed peaks as δ156.3, δ141.9, δ128.7, δ127.4, δ126.3, δ79.9, δ58.9, δ51.5, δ39.3, δ28.2.

**Compound(XIII):**

**The 1H-NMR** (300MHz, CDCl₃) of compound (XIII) showed peaks as δ_{H} 7.38-7.15 (5H, m, Ar), δ3.86 (3H, br s, NH₂, OH), δ3.69-3.63 (3H, m, OCH₂, CH-N), δ2.32-1.77 (2H, m, CCH₂C).

**The 13C-NMR** (75.5 MHz, CDCl₃) of compound (XIII) showed peaks as δ128.9, δ128.6, δ126.9, δ126.2, δ58.9, δ55.1, δ 35.9.

**Compound(XIV):**

**The 1H-NMR** (300MHz, CDCl₃) of compound (XIV) showed peaks as δ_{H} 7.32-7.15 (5H, m, Ar), δ 5.16 (1H, br s, OH), δ 3.90-3.80 (2H, m, OCH₂), δ 3.80-3.69 (1H, m, CH-N), δ 2.48- 2.32 (1H, m, CCH₂ 0C), δ 2.18 (6H, s, N(CH₃)₂), δ 1.76-1.66 (1H, m, CCH₂C);

**The 13C-NMR** (75.5 MHz, CDCl₃) of compound (XIV) showed peaks as δ 136.1, δ 129.0, δ 128.8, δ 128.3, δ127.9, δ 127.1, δ 70.0, δ 63.1, δ 41.0, δ 32.

**Compound(XV):**

**The 1H-NMR** (300MHz, CDCl₃) of compound (XV) showed peaks as δ_{H} 8.27-8.24 (m, 1H, Ar), δ 7.81-7.78 (m, 1H, Ar), δ 7.58-7.51 (m, 2H, Ar), δ 7.47-7.31 (m, 7H, Ar), δ 6.73-6.70 (m, 1H, Ar), δ 4.18-4.01 (m, 1H, H1), δ 3.99-3.93 (m, 1H, H1), 3.71-3.66 (m, 1H, H3), δ 2.77-2.65 (m, 1H, H2), δ 2.40-2.31 (m, 1H, H2),δ 2.32 (s, 6H, H4+H40 );

**The 13C-NMR** (75.5 MHz, CDCl₃) of compound (XV) showed peaks as δ 154.5, δ139.3, δ134.3, δ132.1, δ131.9, δ128.5, δ128.3, δ128.2, δ 127.3, δ126.2, δ125.8, δ125.5, δ125.0, δ121.9, δ119.9, δ104.4 (16C, Ar), δ67.6 (1C, C3), δ65.5 (1C, C1), δ42.7 (2C, C4+C40), δ32.9 (1C, C2).

### Example

The present invention is illustrated with the following example, which should not be construed as limiting the scope of the invention.

**1.Preparation of N-(4-methoxyphenyl)benzaldimine (III)**

A solution of benzaldehyde in dichloromethane is added 1.1 equiv of *p* -anisidine and large excess of anhydrous magnesium sulphate and stirred at room temperature for 12 hours. The yellowish slurry was filtered and concentrated under reduced pressure and the crude imine was taken to the next step without purification (yield 90%).

**2.Preparation of (±)1-(p-Metoxyphenyl)-3-acetoxy-4-phenyl azetidine-2-one (V)**

To the crude imine dissolved in anhydrous dichloromethane was added 3 equiv of triethylamine (Hünig's base improved yields) and cooled to -78 ° C. To this solution, 1.2 equivalents of acetoxyacetyl chloride were added dropwise and the thick reaction mixture was allowed slowly to warm-up to room temperature and stirred for 12 h. The dark crude reaction mixture was concentrated without aqueous work-up and purified by a normal phase column chromatography (4:6, EtOAc:hexanes). Shorter column chromatograph was done to get sufficiently pure lactams (yield 80%).

**3. Preparation of (-)-cis-1-(p-Metoxyphenyl)-3-hydroxy-4-phenyl-azetidin-2-one(VII)**

The racemic β-lactam was then dissolved in acetonitrile and to this solution phosphate buffer at pH 7.2 was mixed and stirred vigorously. Immobilized Lipase PS Amano enzyme (one to one weight ratio to the lactam) was added and stirred for 2-3 days. The progress of the reaction was monitored by TLC, and after the completion of the reaction, the lipase was filtered off and the solution was worked-up in the usual way. Purification by column chromatography (4:6 EtOAc:hexanes) gave the less polar, enantiomerically pure acetoxy-β-lactam (48-50% yield based on the required isomer).

The solution of acetoxy-β-lactam in THF was added slowly to a 1 M KOH solution at 0°C. The solution was stirred 45 min and the deacetylation reaction was completed. The THF in the reaction mixture was evaporated under reduced pressure and at low temperature and diluted with ethyl acetate and worked-up in the usual way. The product of the reaction was used for the next step without purification (quantitative yield).

**4. Preparation of (3S, 4R) Dithiocarbonic acid O-[1-(4-methoxy-phenyl)-2-oxo-4-phenyl-azetidin-3-yl] ester S-methyl ester. (VIII)**

To a cooled suspension of NaH (60%; 0.47 g, 11.8 mmol) in anhydrous THF (5 mL) was added 3-hydroxy-b-lactam 8 (0.80 g, 2.97 mmol) as a solution in THF (5 mL) slowly. After the addition was complete, the reaction mixture was stirred at room temperature for 30 min. The solution was cooled to 0 °C and a solution of CS₂ (0.53 mL, 8.91 mmol) in THF (5 mL) was added. The reaction mixture was stirred for 1.5 h at 0 °C, Mel (1.10 mL, 17.8 mmol) was then added at the same temperature, and the reaction mixture was stirred at room temperature for 3 h. After the reaction was complete (TLC), a saturated aq solution of NH₄Cl (10 mL) was added, and THF was removed under reduced pressure. The residue was dissolved in CH₂Cl₂ (50 mL) and the organic layer was washed with H₂O (20 mL), brine (20 mL), and dried over Na₂SO₄. The solvent was removed under reduced pressure to afford the crude product, which was then purified by flash column chromatography (10% EtOAc/pet. ether) to furnish compound 9 (0.79 g, 75%) as a white solid.

The melting point is 135 °C.

**5. Preparation of (S)-1-(4-Methoxy-phenyl)-4-phenyl-azetidin-2-one (IX)**

A solution of Bu₃SnH (0.58 mL, 2.15 mmol) and AIBN (15 mg) in anhydrous toluene (5 mL) was added drop wise to a refluxing solution of xanthate (0.26 g, 0.72 mmol) in anhydrous toluene (10 mL) under argon atmosphere. The reaction mixture was then refluxed for 3 h (TLC). The solvent was removed under reduced pressure and the residue was purified by flash column chromatography (10% EtOAc/pet. ether) to afford 10 (0.168 g, 92%) as a white fluffy solid.

The melting point is 98°C.

**6. Preparation of (S)-4-Phenyl-azetidin-2-one(X)**

A solution of (NH4)₂Ce(NO3)6 (0.97 g,1.77 mmol) inwater (7 mL) was added drop wise to a solution of (S)-1-(4-Methoxy-phenyl)-4-phenyl-azetidin-2-one (10) (0.15 g, 0.59 mmol) in acetonitrile (7 mL) at 0°C. The mixture was stirred at this temperature for 1 h. Water (10 mL) was added, it was extracted with ethyl acetate (3x15 mL), and washed with saturated solution of NaHCO₃ (2x10 mL). The aqueous layer of NaHCO₃ was extracted again with ethyl acetate (1x10 mL), and the combined organic extracts were washed with 40% NaHSO₃ (3x10 mL) and brine (10 mL). It was dried over anhydrous Na₂SO₄ and the solvent was removed under reduced pressure to get crude product, which was purified by flash column chromatography (70% EtOAc/pet. ether) to furnish (0.052 g, 60%) as a very viscous liquid.

**7.Preparation of (S)-2-Oxo-4-phenyl-azetidine-1-carboxylic acid tert-butyl ester (XI)**

(Boc)₂O (0.94 mL, 4.08 mmol) and DMAP (0.398 g, 3.26 mmol) were added to a solution of azetidin-2-one 11 (0.400 g, 2.72 mmol) in CH₂Cl₂ (10 mL) at 0°C, and the reaction mixture was stirred for 6 h. Then, CH₂Cl₂ (10 mL) was added, and it was washed with a saturated solution of NaHCO₃ (5 mL) and brine (5 mL). The organic layer was dried over anhydrous Na₂SO₄ and the solvent was removed under reduced pressure to get crude product, which was purified by column chromatography (20% EtOAc/pet. ether) to furnish title b-lactam 12 (0.470 g, 70%) as a viscous liquid.

**8.Preparation of S-(3-Hydroxy-1-phenyl-propyl)-carbamic acid tert-butyl ester (XII)**

To a suspension of LAH (0.204 g, 5.37 mmol) in THF (5 mL) was added b-lactam 12 (0.332 g, 1.33 mmol) in THF (5 mL) drop wise at 0°C under inert atmosphere. The reaction mixture was allowed to attain room temperature and stirred for total 4 h. After completion of reaction (TLC), a saturated solution of Na₂SO₄ was added to the reaction mixture at 0 °C and itwas stirred for an hour. THF was then evaporated on rotary evaporator and to the residue was added ethyl acetate (15 mL). It was washed with water (10 mL). The aqueous layer was washed with ethyl acetate (2x5 mL). Combined organic extracts were washed with brine (5 mL) and dried over anhydrous Na₂SO₄ and concentrated in vacuo to get the crude product, which was purified by column chromatography using (40% EtOAc/pet. ether) to furnish 13 (0.269 g, 80%) as a white solid.

The melting point is 104°C.

**9.Preparation of S)-3-Amino-3-phenyl-propan-1-ol (XIII)**

To a solution of (XII) (0.030 g, 0.12 mmol) in DCM (2 mL) was added TFA (0.3 mL) drop wise at 0 °C. It was kept at 0 °C for half an hour and then allowed to come to room temperature and stirred for further 1.5 h. After completion (TLC), the reaction mixture was concentrated in vacuo to remove DCM and TFA. The residue was then dissolved in methanol (3 mL) and Et₃N (0.016 mL, 0.12 mmol) was added and the resultant solution was passed through a short column of silica gel with methanol as an eluent. The methanol fractions were concentrated in vacuo to furnish 14 as a white hygroscopic solid (0.016 g, 89 nmax)

**10.Preparation of (S)-3-Dimethylamino-3-phenyl-propan-1-ol (XIV)**

To a solution of (XIII) (0.12 g, 0.807 mmol) in acetonitrile was added, 30% aq formaldehyde solution (0.325 mL) followed by sodium cyanoborohydride (0.081 g, 1.29 mmol) and it was allowed to stir at room temperature. A few drops of glacial acetic acid were added to maintain the pH near neutrality. The solution was stirred at room temperature for 2 h. After completion of the reaction (TLC), the reaction mixture was concentrated in vacuo. To the residue was added 2 N aq KOH (10 mL). It was then extracted with ethyl acetate (3x10 mL). The ethyl acetate layer was then washed with 1 N HCl (3x5 mL). The combined HCl extracts were basified with solid KOH and then extracted with ethyl acetate (3x10 mL). Combined organic extracts were washed with brine (10 mL), dried over anhydrous Na₂SO₄, and concentrated in vacuo to afford crude product, which was purified by column chromatography (40% EtOAc/MeOH) to furnish 15 (0.115 g, 80%) as a hygroscopic solid.

**11.Preparation of (S)-Dapoxetine**

To a solution of (S)-(XIV) (30 mg, 0.17 mmol) in dry THF (2.4 mL) under nitrogen atmosphere was added 1-naphthol (49 mg, 0.34 mmol). The mixture was cooled to 0°C and PPh3 (89 mg, 0.34 mmol) and DEAD (53.5 IL, 0.34 mmol) were successively added. The solution was allowed to warm until room temperature and stirred during 15 h. The solution was evaporated and the crude purified by flash chromatography (gradient eluent 100% EtOAc to 10%MeOH/EtOAc) isolating 37 mg of a colourless oil (72% isolated yield). Rf (20% MeOH/EtOAc).

## Claims

1. A process for preparing (3S,4R)-3-hydroxy-1-(4-methoxyphenyl)-4-phenylazetidin-2-one, an intermediate of S-(+)-N,N-dimethyl-a-[2-(naphthalenyloxy)ethyl] benzenemethanamine; which process comprises the following steps;(a) reacting benzaldehyde with *p*-anisidine to form N-(4-methoxyphenyl)benzaldimine of formula(III); 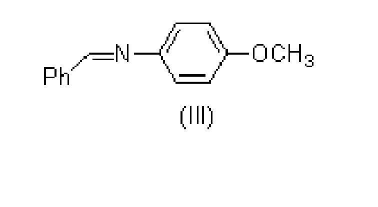
(b) reacting N-(4-methoxyphenyl) benzaldimine with acetoxyacetyl chloride to provide racemic 4-aryl substituted β-lactam derivative of formula(V); 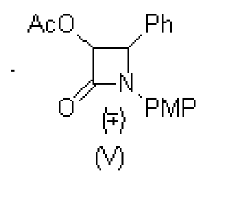
(c) converting 1-(p-methoxyphenyl-3-acetoxy-4-phenylazetidin-2-one into its single enantiomer product via lipase-enzyme catalysed resolution.

2. According to claim 1, (3S,4R)-3-hydroxy-1-(4-methoxyphenyl)-4-phenylazetidin-2-one is prepared by lipase-enzyme catalysed resolution for preparation of S-(+)-N,N-dimethyl-a-[2-(naphthalenyloxy)ethyl] benzenemethanamine.

3. According to claim 1, a process for preparing enantiomerically pure S-(+)-N,N-dimethyl-a-[2-(naphthalenyloxy)ethyl] benzenemethanamine from (3S, 4R)-3-hydroxy-1-(4-methoxyphenyl)-4-phenylazetidin-2-one comprises further following steps; (d)converting the 3-hydroxy b-lactam derivative(VII) to its xanthate derivative(VIII) 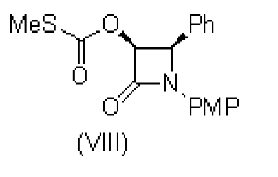
(e) subjecting xanthate derivative of formula (VIII) to deoxygenation to furnish formula (IX). 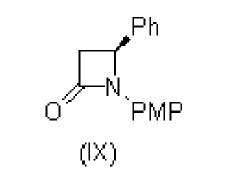
(f) subjecting lactam's p-methoxy-phenyl group to oxidative removal to obtain the compound (X). 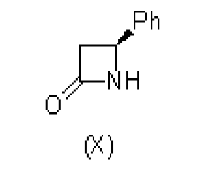
(g) protecting lactam (X) as its carbamate derivative with (BOC)₂O to form the compound(XI). 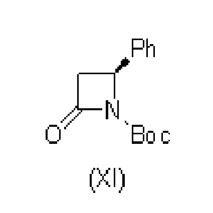
(h) reducting of the compound (XI)by LAH to get BOC protected amino alcohol, namely compound (XII). 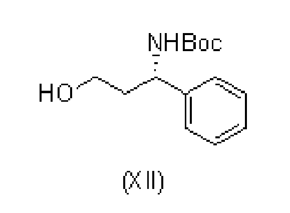
(i)deprotecting of the compound (XII) with TFA to get amino-alcohol compound (XIII). 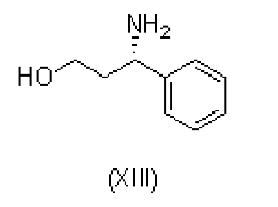
(j) demethylating of the compound (XIII) afforded the desired N- bismethylated amino alcohol of formula (XIV). 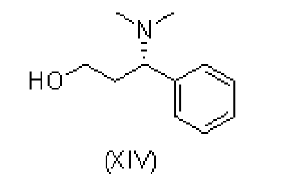
(k) obtaining of enantiomerically pure (s)-dapoxetine
